# EUROPÄISCHE PATENTSCHRIFT

(11) **EP 2 037 853 B1**
(45) Veröffentlichungstag und Bekanntmachung des Hinweises auf die Patenterteilung: **06.06.2012**
(21) Anmeldenummer: 07724252.7
(22) Anmeldetag: 14.04.2007
(51) Int. Cl.: A61F 13/08

(54) **KOMPRESSIONS- ODER STÜTZSTRUMPF**
COMPRESSION OR SUPPORT STOCKING
BAS DE COMPRESSION OU DE CONTENTION

(30) Priorität: 07.07.2006 DE 102006032223
(43) Veröffentlichungstag der Anmeldung: 25.03.2009
(73) Patentinhaber: Paul Hartmann AG, 89522 Heidenheim (DE)
(72) Erfinder: VIRKUS, Antje, 67000 Strasbourg (FR); BERNDT, Erik, 89542 Herbrechtingen (DE)
(74) Vertreter: Langöhrig, Angelika Beate
(86) Internationale Anmeldenummer: PCT/EP2007/003314
(87) Internationale Veröffentlichungsnummer: WO 2008/003361

(56) Entgegenhaltungen:
- WO-A-01/00118
- US-A- 3 392 553
- US-A1- 2002 172 781
- US-A1- 2005 015 854
- US-A1- 2005 267 393

## Beschreibung

Die Erfindung betrifft einen Kompressions- oder Stützstrumpf zum Anlegen an ein menschliches Bein umfassend einen ersten Unterstrumpf und einen ersten Überstrumpf mit je einem Schaftbereich, die übereinander anlegbar sind. Der Überstrumpf wird dabei ständig oder nur zeitweise über dem Unterstrumpf getragen.

Kompressions- und Stützstrümpfe werden zum einen in der medizinischen Kompressionstherapie und zum anderen im nichttherapeutischen, sondern kosmetischen Bereich als Stützstrümpfe verwendet, um einen Ruhe- sowie einen Arbeitsdruck auf ein menschliches Bein aufzubringen. Unter dem Ruhedruck wird dabei der Druck verstanden, den der Strumpf als solcher auf das ruhende Bein aufbringt, wobei der Arbeitsdruck der Druck ist, der beim Bewegen des Beins durch die Kontraktion des Muskels und den Strumpf auf das Bein ausgeübt wird.

Dabei ist es neben der reinen Kompressionstherapie zur Behandlung einer Vielzahl von venösen Leiden auch bekannt, Krankheiten zu behandeln, bei denen gleichzeitig zu einer Wundversorgung auch ein Kompressionsdruck aufgebracht werden soll. Dabei wird zunächst eine Wundauflage auf eine Wunde aufgebracht und dann ein Kompressionsstrumpf hierüber gezogen.

Zur Behandlung derartiger Erkrankungen ist beispielsweise aus der US-PS 5,005,567 eine Methode bekannt, wobei ein locker gestrickter Socken, der leicht übergezogen werden kann, zunächst über das Wundverbandsmaterial gezogen wird und dann ein elastischer Socken mit einem geringen Reibungskoeffizienten zwischen den Strümpfen über den ersten Unterstrumpf gezogen wird, wobei das Anziehen des zweiten Strumpfes erleichtert ist aufgrund der geringen Reibung zwischen den Strümpfen.

Dabei ist vorgesehen, dass der Unterstrumpf keinerlei kompressive Wirkung aufweist. Der Überstrumpf kann hier eine Kompression von *1333,22 bis 6666,1 Pa* (10 bis 50 mm Quecksilber) im Bereich des Knöchels aufweisen.

Nachteilig hierbei ist, dass der Überstrumpf eine hohe Kompressionskraft aufbringen muss, da sämtliche Kompression durch den Überstrumpf erzielt werden muss. Darüber hinaus besteht die Gefahr, dass es durch das lose Anlegen des Unterstrumpfes zur Faltenbildung im Bereich des Unterstrumpfes kommt.

Des Weiteren ist es aus der DE 42 16 650 A1 zur Behandlung kosmetisch relevanter Orangenhaut und zur Verbesserung der physiologischen Muskelpumpe bekannt, einen kosmetischen Stützstrumpf vorzusehen, der aus mehreren Kompressionsstrümpfen oder Kompressionsstrumpfhosen oder Bandagen besteht, die übereinander anzulegen sind. Darüber hinaus können die einzelnen Teile fest oder variabel miteinander verbunden werden, um diese zueinander zu sichern.

Dabei hat jeder der Strümpfe für sich nur eine geringe Kompressionswirkung und lässt sich daher mit Leichtigkeit und schnell anlegen. Dadurch, dass mehrere Strümpfe übereinander gezogen werden, erhöht sich der Druck auf die Haut jedoch wesentlich, so dass letztendlich ein ausreichender Kompressionsdruck entsteht.

*Aus der WO 01*/*00118 A1 ist ein mehrlagiges Kompressionsstrumpfsystem bekannt, bei dem Markierungen für die Positionierung der Strümpfe aufeinander vorgesehen sind, um die richtige Anordnung des kompressiven Drucks an einem Bein zu erreichen.*

*Aus der US 2002*/*172781 A1 ist ein kompressiver elastischer Strumpf aus zwei Teilen, nämlich einem Fuß- und einem Wadenteil bekannt, die sich im Bereich des Knöchels überlappen. Hierdurch soll eine bessere Anziehbarkeit eines bis zum Knie reichenden Kompressionsstrumpfs gegeben sein.*

*Die* US 3,392,553 *betrifft einen halterlosen Damenstrumpf, der im Bereich der Bündchen sowohl gewisse Kompressions- als auch Reibungskräfte aufweisen kann, um den Strumpf am Bein des Trägers zu halten.*

Ferner sind eine Vielzahl von einlagigen Kompressionsstrümpfen vorbekannt.

Für die Messung und zur Gütesicherung für medizinische Kompressionsstrümpfe existiert die Norm RAL-GZ 387 vom September 2000. Aus den Prüfbestimmungen der Norm kann entnommen werden, wie der Druck eines Kompressionsstrumpfes auf ein Bein zu bestimmen ist.

Der Erfindung liegt nun die Aufgabe zugrunde, einen gattungsgemäßen Kompressions- oder Stützstrumpf zum Anlegen an ein menschliches Bein bereitzustellen, der zur Behandlung von chronischer venöser Insuffizienz sowie deren Erscheinungen und Folgeerkrankungen, wie beispielsweise der Ulcus cruris venosum, vorteilhaft einsetzbar ist und dennoch eine leichte Anziehbarkeit gewährleistet.

Die Erfindung löst diese Aufgabe durch einen Kompressions- oder Stützstrumpf gemäß den Merkmalen des Anspruchs 1, bei dem der Unterstrumpf im Bereich zwischen den Messstellen B und B1 einen kontinuierlichen Druckverlauf aufweist und der Druck an der Messstelle B1 90 bis 100% des Drucks an der Messstelle B entspricht, *wobei im Schaftbereich von Unter- und Überstrumpf ein in Umfangsrichtung verlaufendes Wellenmuster so eingestrickt ist, dass sich erhabene und weniger erhabene Zonen bilden, wobei die Muster von Unter- und Überstrumpf im übereinandergezogenen Zustand deckungsgleich sind.*

Bei den Messstellen B und B1 handelt es sich um Stellen, die den Fesselbereich eines menschlichen Beins betreffen und diesen zwischen sich einschließen. Die Messstellen werden gemäß der heranzuziehenden Vorschrift RAL-GZ 387 bestimmt. Unter einem kontinuierlichen Druckverlauf zwischen den Messstellen B und B1 ist im Zusammenhang mit der vorliegenden Erfindung ein Druckverlauf zu verstehen, dessen interpolierte Einzelwerte an jedem Punkt zwischen den Punkten B und B1 höchstens um 10%, insbesondere um höchstens 7% und ganz besonders um höchstens 5 % von dem Messwert an B abweichen. Bei einem kontinuierlichen Druckverlauf zwischen anderen Messstellen ist die vorliegende Maßgabe entsprechend anzuwenden.

Dabei entspricht die Messstelle B einem Punkt am menschlichen Unterschenkel, der unmittelbar über dem Knöchel an dem Punkt mit dem geringsten Umfang des Unterschenkels liegt. Die Messstelle B1 entspricht einem Punkt am menschlichen Unterschenkel, der im vorgegebenen Abstand darüber, also proximal beim Übergang der Achillessehne zur Wade, angeordnet. Weitere Punkte gemäß der RAL-Norm (RAL-GZ 387) sind beispielsweise der Punkt C, nämlich der größte Umfang der Wade, sowie der Punkt D, der zwei Finger breit unterhalb der Kniekehle liegt.

Dabei ist vorgesehen, dass an der Messstelle B1 im Unterstrumpf der Druck zwischen 90 und 100% des Drucks an der Messstelle B entspricht. Vorzugsweise ist der Druck an der Messstelle B gleich dem Druck an der Messstelle B1. Der Bereich zwischen den Messstellen B und B1 ist als Therapiebereich insbesondere zur Behandlung von Folgeerkrankungen der chronischen venösen Insuffizienz wie beispielsweise des Ulcus cruris venosum, der in seiner schwersten Form als Gamaschenulkus bekannt ist, vorgesehen, wobei der Patient im Bereich der Fessel offene Hautläsionen aufweist. Auf diese werden nun zur Behandlung Wundauflagen aufgebracht, die dann durch den Unterstrumpf überdeckt sind. Durch die Vorsehung eines im Wesentlichen einheitlichen bzw. lediglich höchstens 10% abweichenden Druckverlaufes im Bereich des Knöchels wird erreicht, dass über den gesamten Therapiebereich ein konstanter Druck aufgebaut wird, so dass die zu behandelnde Hautoberfläche nicht durch unterschiedliche Drücke belastet ist.

Damit betrifft die vorliegende Erfindung auch die Verwendung eines Kompressions- oder Stützstrumpfes zum Anlegen an ein menschliches Bein umfassend einen ersten Unterstrumpf und einen ersten Überstrumpf mit je einem Schaftbereich und einem Fußbereich, die übereinander anlegbar sind, wobei der Unterstrumpf im Bereich zwischen den Messstellen B und B1 einen kontinuierlichen Druckverlauf aufweist und der Druck an der Messstelle B1 90-100% des Drucks an der Messstelle B entspricht, zur Herstellung eines Mittels zur Behandlung von chronischer venöser Insuffizienz sowie deren Erscheinungen und Folgeerkrankungen, insbesondere abgeheiltes oder florides Ulcus cruris venosum, Dermatoliposklerose und/ oder Atrophie blanche.

Grundsätzlich kann die chronische venöse Insuffizienz nach Widmer in drei Stadien eingeteilt werden. Demnach wird die chronische venöse Insuffizienz unterschieden in:
Grad I der chronischen venösen Insuffizienz ist demnach durch um die Knöchel und oberhalb des Fußgewölbes angeordnete, besenreiserartige Venen (Corona phlebectatica) und Knöchelödeme gekennzeichnet.
Grad II äußert sich durch Hyperpigmentierungen der Haut, Unterschenkelödem und Dermatoliposklerose. Die Haut ist fest mit der Fascia cruris verbacken, nicht in Falten abhebbar und zeigt vermehrten Glanz. Als Extremvariante der Dermatoliposklerose gilt die Atrophie blanche (auch als Capillaritis alba bezeichnet), die fast ausschließlich als Folge einer chronischen venösen Insuffizienz vorkommt und sich durch Depigmentierung im distalen Unterschenkel infolge Vaskulitis kleiner Hautgefäße zeigt, die oft eine schmerzhafte Vorstufe des Ulcus cruris ist. Charakteristisch für diese Hautveränderung sind weiße, atrophische, münz- bis handtellergroße Herde. Sie sind bevorzugt in der Knöchelregion bzw. im Narbenbereich abgeheilter Ulzerationen lokalisiert.
Grad III manifestiert sich als florides oder abgeheiltes Ulcus cruris venosum. Es hat als Prädilektionsstelle die perimalleoläre Region (Bisgaard'sche Kulisse), kann jedoch auch an anderen Stellen am Unterschenkel auftreten. Bei ausgedehnten Geschwüren, die zirkulär den gesamten Unterschenkel befallen, spricht man von einem Gamaschenulkus. Eine weitere heute geläufige Einteilung der chronischen venösen Insuffizienz erfolgt nach der CEAP-Klassifikation. Hierzu sei auf die Publikation "Grundlagen der Phlebologie, Kapitel 4.3 Chronische Venöse Insuffizienz (Herausgeber R. Rabe, 3. Auflage, S. 111 - 129) verwiesen.

Insbesondere kann vorgesehen sein, dass der Überstrumpf und/oder der Unterstrumpf eines erfindungsgemäßen Kompressions- oder Stützstrumpfes als Gestrick, vorzugsweise Rundgestrick ausgestaltet ist. Insbesondere werden die Strümpfe als sogenannte AD-Strümpfe gemäß RAL-GZ 387 gefertigt. Auch können die Strümpfe gemäß des im "Referentiel technique des ortheses elastiques de contention des membres" (Revision No.05) beschriebenen Pflichtenheftes (Cahier des charges) gefertigt werden. Generell können solche Strümpfe einen fakultativen Zehen- und einen Fußteil mit Ferse, einen hieran anschließenden Schaft im Bereich des Unterschenkels und einen den Strumpf abschließenden Bund aufweisen.

Vorzugsweise ist dabei vorgesehen, dass der Unterstrumpf an der Messstelle B1 zwischen 93 und 100%, insbesondere 95 bis 100% und insbesondere 98 bis 100% des Drucks an der Messstelle B aufweist. Durch den möglichst konstanten Druckverlauf wird die Wundheilung und auch die Fixierung der Wundauflage unter dem Unterstrumpf verbessert.

Weiterhin kann vorgesehen sein, dass der Überstrumpf ausgehend von der Messstelle B in Richtung der Hüfte des menschlichen Beins, also nach proximal, einen stufenlos degressiven oder stufenweise degressiven Druckverlauf aufweist. Insbesondere kann es sich bei dem Überstrumpf um einen Kompressionsstrumpf mit einem Druckverlauf gemäß der RAL-Norm GZ 387 handeln.

Dabei sind gemäß RAL-Norm (RAL-GZ 387) verschiedene Kompressionsklassen vorgesehen, wobei der Überstrumpf ein Strumpf der Kompressionsklassen 1 bis 4 gemäß Norm sein kann.

Bei dem Unterstrumpf kann es sich insbesondere um einen Strumpf handeln, der hinsichtlich des Druckverlaufs nicht der RAL-Norm entspricht, wobei jedoch auch Strümpfe gemäß der Norm vorgesehen sein können. Der Unterstrumpf weist im Bereich proximal der Messstelle B1 einen degressiven Verlauf auf, der stufenlos oder stufenweise degressiv verläuft, oder einen kontinuierlichen bzw. gleichbleibenden Druckverlauf auf. Wenn vorgesehen ist, dass der Unterstrumpf im Bereich proximal der Messstelle B1 bzw. proximal des Therapiebereichs einen kontinuierlichen Druckverlauf aufweist, dann kann insbesondere auch vorgesehen sein, das der Unterstrumpf lediglich einen geringen Kompressionsdruck von weniger als 8 mm Hg aufbringt. Eine Faltenbildung unter dem Überstrumpf beim Anziehen desselben muss dennoch verhindert werden.

Dabei ist insbesondere vorgesehen, dass der Unterstrumpf für die Bereitstellung des gewünschten Ruhe- und Arbeitsdruck verantwortlich ist, wobei der Überstrumpf einen zusätzlichen Beitrag hierzu liefert. Daher kann besonders vorteilhaft vorgesehen sein, wenn der Unterstrumpf Tag und Nacht getragen wird, um die Wundauflage sicher auf der zu behandelnden Wunde zu fixieren und ein zu häufiges Ablösen der Wundauflage von der Wunde zu verhindern, wodurch die Wundheilung beeinträchtigt wird. Gleichzeitig wird jedoch hierdurch erreicht, dass zum Zeitpunkt der Nachtruhe, während derer die Beine normalerweise hochgelegt sind, lediglich eine schwache Kompressionswirkung erzielt wird, so dass es nicht zu einem Taubheitsgefühl oder Abschnürungen im Beinbereich kommt. Im Verlauf des Tages, während dessen sich der Träger des Kompressionsstrumpfes bewegt, kann dann zusätzlich ohne Störung der Wundheilung der Überstrumpf über den Unterstrumpf gezogen werden, der eine stärkere Kompressionswirkung bereitstellt, so dass dann im Verlauf des Tages zusätzlich zum bereits bestehenden Ruhe- und Arbeitsdruck durch den Unterstrumpf ein vergleichbarer stärkerer Ruhe- und Arbeitsdruck durch den Überstrumpf erzielt wird, so dass eine zusätzliche Kompressionstherapie durch die geleistete Muskelarbeit erfolgt und so das Grundleiden positiv beeinflusst wird. Durch die Vorsehung von zwei übereinanderliegenden Strümpfen kann jeder Strumpf nur eine geringere Kompressionswirkung haben, wodurch das Anziehen erleichtert wird.

Dabei kann vorgesehen sein, dass der Kompressionsdruck von Ober- und Unterstrumpf in der Größe zusammen *666,61 bis 1999,83 Pa* (5 bis 15 mm Quecksilber mm Hg) über dem Kompressionsdruck der Einzelstrümpfe liegt. Die Messung des Kompressionsdrucks erfolgt hierbei mittels eines Kompressionsprüfgerätes nach dem System Hohenstein (Hosy), wobei eine detaillierte Beschreibung des Prüfverfahrens sich aus dem Hohensteiner Forschungsbericht vom Januar 1982 und Phlebol und Proktol. 11: 34-41 (1982) entnehmen lässt.

Dabei ist vorgesehen, dass die Kompression am Punkt B beider Strümpfe zusammen zwischen *4532,95 und 7999,32 Pa* (34 und 60 mm Hg), vorzugsweise zwischen *4532,95 und 6666,1 Pa* (34 und 50 mm Hg) liegt, wobei ein mm Hg 133, 322 Pascal entspricht.

Dabei weist der Unterstrumpf im Messpunkt B eine Kompression von *1999,83 - 4266,3 Pa* (15 bis 32 mm Hg) und vorzugsweise von *1999,83 - 3333,05 Pa* (15 bis 25 mm Hg) auf. Im Punkt B1 besteht ein Wert von 90 bis 100% des Wertes am Punkt B. In einem Punkt C können Werte von 5 bis 80%, vorzugsweise zwischen 50 und 80% oder 5 bis 50% des Drucks am Punkt B vorliegen, wobei die Druckabnahme zwischen B1 und C erfolgt.

Für den Überstrumpf, der nach der RAL-Norm (RAL-GZ 387) gefertigt ist, liegt am Punkt B eine Kompression von *1999,83 - 4266,3 Pa* (15 bis 32 mm Hg) vor.

Es kann auch vorgesehen sein, dass der Arbeitsdruck, der durch das Tragen eines erfindungsgemäßen Kompressions- oder Stützstrumpfes auf ein Bein hervorgerufen wird gegenüber dem Ruhedruck mindestens *1333,22 - 4666,27 Pa* (10 bis 35 mm Hg) größer ist, wobei der Ruhedruck mindestens *4532,95 Pa* (34 mm Hg) beträgt.

Insbesondere kann es sich dabei bei den Kompressionsstrümpfen gemäß der Erfindung um rundgestrickte Kompressionsstrümpfe handeln, wobei insbesondere sogenannte AD-Strümpfe, nämlich Wadenstrümpfe gemäß der RAL-Norm (RAL-GZ 387), vorgesehen sind. Es kann auch vorgesehen sein, dass die Kompressionsstrümpfe gemäß des im "Referentiel technique des ortheses elastiques de contention des membres" (Revision No.05) beschriebenen Pflichtenheftes (Cahier des charges) gefertigt werden.

Um ein leichteres Anziehen des Überstrumpfes über den Unterstrumpf zu gewährleisten, kann des Weiteren vorgesehen sein, dass der Unterstrumpf einen angeformten Fußteil aufweist, der einen dynamischen Reibungskoeffizienten von µ_{D} ≤ 0,65, insbesondere µ_{D} ≤ 0,60 und ganz besonders bevorzugt µ_{D} ≤ 0,55 besitzt. Der Reibungskoeffizient wird dabei analog der DIN-EN-ISO 8295: 2004-10 gemessen. Hierbei wird das zu prüfende Material des angeformten Fußteils gegen das Material vermessen, das im anwendungsgerechten Zustand der Strumpfes auf der Innenseite eines Überstrumpfes liegt und zwar die Oberfläche (Gegenmaterial), die im anwendungsgerechten Zustand über den Fußteil zu ziehen ist. Die Materialien werden dabei im nicht gedehnten Zustand untersucht, wobei sicherzustellen ist, dass alle Materialien glatt liegen, die Materialien längs ihrer Herstellrichtung vermessen werden und das Gegenmaterial längs seiner Herstellrichtung eingespannt ist. Ansonsten sind die Bedingungen der oben zitierten Norm einzuhalten, wobei insbesondere eine Prüfungsgeschwindigkeit von 100 mm/ min einzuhalten ist.

Der Fußteil des Unterstrumpfes kann einen angestrickten Zehenbereich aufweisen sowie eine angeformte Ferse, wobei die Ferse insbesondere keinen Nahtbereich aufweist, sondern durch thermische Verformung des Gewebes erzielt wird. Der Fußbereich ist vorzugsweise kompressionslos, wobei durch das sehr glatte Gewebe eine leichte Anziehbarkeit des Überstrumpfes im stark gekrümmten Fußbereich gewährleistet werden kann.

Der Überstrumpf und/ oder der Unterstrumpf kann eine sogenannte Pendelferse aufweisen, die der RAL-Norm RAL-GZ 387 entspricht, wobei hier der Zehenbereich offen oder geschlossen ausgebildet sein kann.

Insbesondere kann der Unterstrumpf und/ oder der Überstrumpf auch angrenzend an den Schaftbereich einen Druckentlastungsbereich aufweisen. Dieser Druckentlastungsbereich liegt im anwendungsgerechten Zustand des Strumpfes bzw. der Strümpfe im Bereich des Knöchels und ist im Strumpf zwischen dem Schaftbereich und dem Fußbereich angeordnet. Dieser Druckentlastungsbereich hat den Vorteil, dass angrenzend an den Fesselbereich insbesondere am Punkt B, der den höchsten Druck aufweist, kein abrupter Übergang von sehr hohem Druck auf niedrigen bis sehr niedrigen Druck erfolgt und somit keine Abschnürungen innerhalb der Fläche zu verzeichnen sind, die von dem Strumpf bedeckt wird. Ganz besonders bevorzugt ist dabei, wenn der Unter- und/ oder Überstrumpf eine Pendelferse umfasst, wobei der Pendelferse angrenzend ein Druckentlastungsbereich zugeordnet ist. Insbesondere kann dieser Druckentlastungsbereich in Seitenansicht des Strumpfes eine Ypsilon-Form (Y-Form) aufweisen. In dieser Ausgestaltung ist die Pendelferse vollumfänglich von dem Druckentlastungsbereich eingefasst. Es kann jedoch auch vorgesehen sein, dass der Druckentlastungsbereich im Unter- und/ oder Überstrumpf radial am Ende des Schaftbereiches angeordnet ist.

Des Weiteren ist vorgesehen, dass im Schaftbereich von Unter- und/oder Überstrumpf ein Muster, nämlich ein Wellenmuster, wobei die Wellen in Umfangsrichtung verlaufen, eingebracht ist. Dieses Wellenmuster ist in das Material eingestrickt, so dass sich erhabene und weniger erhabene Zonen bilden. Durch dieses Muster kann erreicht werden, dass ein besseres Fixieren der beiden Strümpfe übereinander erfolgt, da hierdurch die Rauhigkeit und damit die Haftung der beiden Schaftbereiche erhöht werden. Dabei ist es besonders bevorzugt, wenn das Muster von Unter- und Überstrumpf im übereinander gezogenen Zustand deckungsgleich zu liegen kommen.

Ebenfalls vorteilhaft ist, wenn der Unterstrumpf eine Markierung zur Positionierung des Überstrumpfes beinhaltet. Diese Markierung kann insbesondere durch die Bündchenkante des Unterstrumpfes gegeben sein. Darüber hinaus kann die Markierung durch einen Farb-, Textur- oder Musterwechsel im Unterstrumpf gebildet sein, wobei bis zu dieser Markierung dann die Oberkante des Schaftes bzw. des Bundes des Überstrumpfes gezogen wird.

Die Gestricke der Strümpfe umfassen dabei vorzugsweise mindestens einen elastischen Einlegefaden und mindestens einen elastischen Strickfaden zur Erzielung der gewünschten elastischen Eigenschaften.

Besonders bevorzugt ist es, wenn der Unter- und/oder der Überstrumpf wenigstens im Bereich des Schaftes Fäden aufweisen, bei denen ein elastischer Kernfaden mit einem Baumwollfasern enthaltenden Garn, insbesondere einem Stapelfasergarn als Umwindefaden, umwickelt ist. Dabei kann besonders vorteilhaft vorgesehen sein, dass als Kernfaden ein Elastankern, beispielsweise ein Kern aus Lycra®, verwendet wird, wobei dieser Kern einfach, doppelt oder mehrfach umwunden sein kann, wobei das zum Umwinden verwendete Garnmaterial zum einen ein Multifilamentgarn aus Polyamid sein kann und zum anderen zum Umwinden sowohl ein Multifilamentgarn aus Polyamid als auch ein Stapelfasergarn verwendet werden kann.

Dabei ist es vorteilhaft, wenn das Stapelfasergarn 70 bis 90% Baumwolle und 10 bis 30% eines Algen enthaltenden Zellulosematerials beinhaltet. Dabei können als Algenmaterial enthaltende Zellulosefasern insbesondere Seacellfasern® (SeaCell GmbH, Rudolstadt, Deutschland) eingesetzt werden, bei denen in die Zellulosefasern Algenmaterialien inkorporiert sind. Die Algen liegen dabei in Partikelform gleichmäßig innerhalb der Zellulosefaser vor.

Als Algenpartikel können hierbei Braunalgen, nämlich vorzugsweise Ascophyllum Nodosum, oder Rotalgen, nämlich vorzugsweise Lithothamnium Calcareum eingesetzt werden. Die Fasern können nach dem Lyocell-Verfahren hergestellt werden. Der Einsatz derartiger Fasern bildet den Vorteil, dass in Gegenwart von Wasser und/oder Schweiß Wirkstoffe wie Vitamin E (Tocopherole), Carotenoide und Mineralien in Form ihrer Ionen, wie z. B. Natrium-, Kalzium- oder Magnesiumionen, freigesetzt werden. Dabei sind die Wirkstoffe in den Algen enthaltenden Fasern längerfristig inkorporiert und werden auch durch Waschvorgänge nicht sofort ausgewaschen. Seacell® oder andere Algen enthaltende Fasern bestehen dabei aus mindestens 90 Gew.-% Zellulose, 0,1-10 Gew.-% Algen und 0-10 Gew.-% weiteren Zusatzstoffen. Die in den Algen enthaltenen Wirkstoffe wirken dabei bei Abgabe positiv auf die Haut des Trägers. Sofern sowohl im Unter- als auch im Überstrumpf Algenmaterial enthalten ist, verhindert dies einen Wirkstoffgradienten zwischen den Strümpfen und fördert so die Abgabe der Wirkstoffe an die Haut des Trägers.

Im Folgenden soll anhand einer Zeichnung die Erfindung näher erläutert werden. Dabei zeigen:
- Figur 1:: ein erfindungsgemäßer Kompressionsstrumpf,
- Figur 2:: ein Legebild eines Teils eines Schaftbereiches eines Unterstrumpfes,
- Figur 3:: ein Legebild eines weiteren Teils eines Schaftbereiches des Unterstrumpfes und
- Figuren 4 und 5:: die Druckmesskurven eines erfindungsgemäßen Kompressionsstrumpfes.

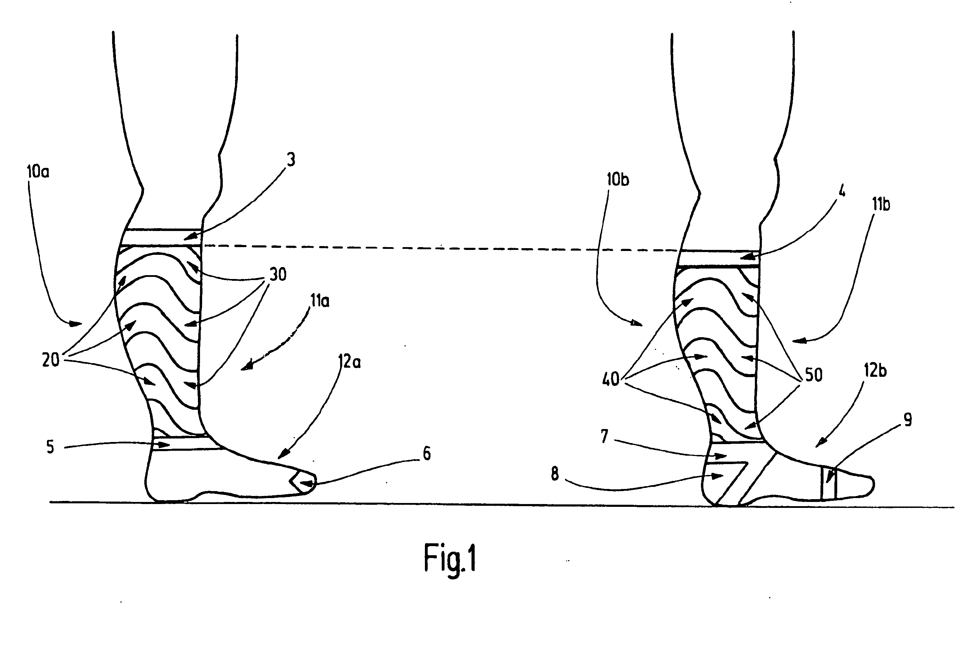

Der Figur 1 lässt sich der Aufbau eines erfindungsgemäßen Kompressionsstrumpfes 10 entnehmen. Dieser Kompressionsstrumpf ist als Kniestrumpf bzw. AD-Strumpf gefertigt und besteht aus einem Unterstrumpf 10a und einem Überstrumpf 10b mit jeweils einem Schaftbereich 11a, 11b und jeweils einem Fußbereich 12a, 12b, die übereinander anlegbar und tragbar sind. Zur Verdeutlichung der Einzelbestandteile jedes Strumpfes sind in Figur 1 der Unterstrumpf 10a sowie des Überstrumpfes 10b an demselben Bein 1 separat dargestellt. Im Gebrauch werden diese Strümpfe derart angezogen bzw. übereinander getragen, dass die Oberkante des Abschlussbundes 4 des Überstrumpfes mit der Unterkante des Abschlussbundes 3 der Unterstrumpfes übereinstimmt (in Figur 1 durch die gestrichelte Linie dargestellt).

Der Unterstrumpf 10a weist am oberen Ende des Fußbereichs 12a, angrenzend zum Schaftbereich 11a einen Druckentlastungsbereich 5 auf. Dieser Druckentlastungsbereich 5 ist nahtlos an den Fußbereich 12a und nahtlos an den Schaftbereich 11a angeformt. Im Unterstrumpf 10a ist der Fußbereich 12a mit einer geschlossenen Spitze 6 ausgeführt, wobei diese Spitze im Vergleich zum übrigen Fußbereich mit einem verstärkten Material ausgeführt ist.

Der Überstrumpf 10b weist ebenfalls einen

Druckentlastungsbereich 7 auf. Dieser Entlastungsbereich 7 umschließt eine hier vorgesehene die Pendelferse 8 vollständig und ist nahtlos an einen Schaftbereich 11a sowie nahtlos an die Pendelferse 8 angeformt. Der Druckentlastungsbereich hat in Abhängigkeit von der Größe der Ferse in der Seitenansicht wie dargestellt eine V-Form oder eine Y-Form (nicht dargestellt). Im Überstrumpf 10b ist der Fußbereich 12b mit einer offenen Spitze ausgeführt, wobei der Fußbereich mit einem Bund 9 abgeschlossen ist.

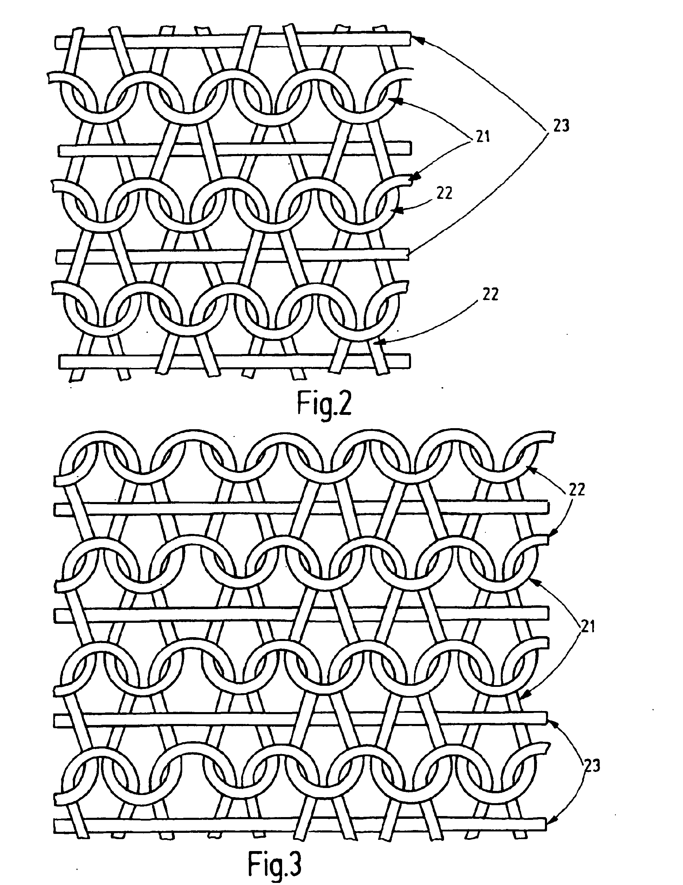

Sowohl der Unterstrumpf 10a als auch der Überstrumpf 10b ist als Rundgestrick ausgeführt, wobei jeder Strumpf im jeweiligen Schaftbereich 11a, 11b mit einem Wellenmuster versehen ist. Dieses Wellenmuster wird durch unterschiedliche Arten des Strickens ermöglicht, wobei zwei verschiedene Strickarten verwendet werden. Das Wellenmuster ist derart ausgebildet, dass jeweils eine Strickweise auf die andere alternierend folgt. Das Strickmuster des Unterstrumpfes 10a im Schaftbereich 11a ist mit den Legebildern in Figur 2 und Figur 3 wiedergegeben. Mit Figur 2 ist das Legebild der Bereiche 20 (Figur 1) wiedergegeben. Demnach werden in diesen Bereichen zwei verschiedene Strickfäden 21, 22 und ein Einlegefaden 23 verwendet. Der Unterstrumpf ist mit einem 1:1-Einlegefaden und wechselndem Strickfaden ausgestattet. In Figur 3 ist die Strickart der Bereiche 30 (s. Figur 1) wiedergegeben. Demnach werden in diesen Bereichen die gleichen Strickfäden 21, 22 (im Vergleich zu Figur 2) und der gleiche Einlegefaden 23 verwendet. Im Unterschied zu den Bereichen 20 wird der Einlegefaden in den Bereichen 30 jedoch als 2:2-Einlegefaden verwendet.

Sowohl der Druckentlastungsbereich 5 des Unterstrumpfes als auch der Druckentlastungsbereich 7 des Überstrumpfes ist aus denselben Materialien gefertigt wie die jeweiligen Schaftbereiche 11a, 11b der beiden Strümpfe. In diesen Bereichen ist der Einlegefaden als 3:2-Einlegefaden verwendet.

Die Legebilder der korrespondierenden Bereiche 40 und 50 im Überstrumpf 10b entsprechen den in Figur 2 und 3 wiedergegebenen Legebildern, mit dem Unterschied, dass im Überstrumpf über den gesamten Schaftbereich 11b nur ein Strickfaden 41 und ein Einlegefaden 42 verwendet wird (hier nicht dargestellt).

Im folgenden Beispiel sind die Materialien für Unter- und Überstrumpf angegeben. Die Nummerierung bezieht sich auf Figur 1, 2 und 3.

### Materialien Überstrumpf 10b:

Strickfäden Bund 4:
a) 44 dtex EL Lycra®(Kern) - doppelt umwunden mit
   i) 44f26/1 dtex PA 6.6 (Tactel® diabolo)
b) 78 dtex EL Lycra®(Kern) - doppelt umwunden mit
   i) 26f28/1 dtex PA 6.6 (Mikrofasergarn)

Strickfaden 41 im Schaft 11b, Fuß 12b und Druckentlastungsbereich 7:
a) 44 dtex EL Lycra@(Kern) - doppelt umwunden mit
   i) 44f26/1 dtex PA 6.6 (Tactel® diabolo)

Einlegefaden 42 im Schaft 11b, Fuß 12b und Druckentlastungsbereich 7:
a) 570 dtex EL Lycra®(Kern) - doppelt umwunden mit
   i) 44f13/1 dtex PA 6.6 und
   ii) Stapelfasergarn Nm170/1

Strickfaden Ferse 8 und Bund 9 (offene Spitze):
a) 78 dtex EL Lycra®(Kern) - doppelt umwunden mit
   i) 60f68/1 dtex PA 6.6 (Mikrofasergarn)

### Materialien Unterstrumpf 10a:

Strickfaden Bund 3:
a) 78 dtex EL Lycra® (Kern) - doppelt umwunden mit
   i) 26f28/1 dtex PA 6.6 (Mikrofasergarn)

Strickfaden 21, 22 im Schaft 11a und Druckentlastungsbereich 5:
a) 44 dtex EL Lycra®(Kern) - doppelt umwunden mit
   i) 44f26/1 dtex PA 6.6 (Tactel® diabolo)
b) 44 dtex EL Lycra®(Kern) - doppelt umwunden mit
   i) 44f34/1 dtex PA 6.6 und
   ii) Stapelfasergarn Nm170/1

Einlegefaden 24 im Schaft 11a und Druckentlastungsbereich 5:
a) 156 dtex EL Lycra®(Kern) - doppelt umwunden mit
   i) 44f13/1 dtex PA 6.6 und
   ii) Stapelfasergarn Nm170/1

Strickfaden im Fuß 12a:
a) 78f51/1 dtex Tactel® PA 6.6
b) 44 dtex EL Lycra®(Kern) - doppelt umwunden mit
   i) 44f26/1 dtex PA 6.6 (Tactel® diabolo)

Strickfaden Spitze 6:
a) 78 dtex EL Lycra®(Kern) - doppelt umwunden mit
   i) 26f28/1 dtex PA 6.6 (Mikrofasern)

EL steht dabei für Elastan und Nm für Nummer metrisch. Ferner wird ein doppelt umwundener Elastankern mit einem ersten Garn i) als erste Lage umwunden und mit einem zweiten Garn ii) als zweite Lage oder mit dem ersten Garn i) als zweite Lage über die erste Lage umwunden, wobei die Fäden verkreuzt angeordnet sind. Garnlieferant für die umwundenen Garne ist Firma Zimmermann, Weiler Simmerberg, Deutschland. Die Zusammensetzung des Stapelfasergarns ist wie folgt: 75% Baumwolle, 25% Seacell®, Seacell® enthält 8 bis 12% Algenanteil in Zellulose. Das Garn stammt von der SeaCell GmbH, Rudolstadt, Deutschland.

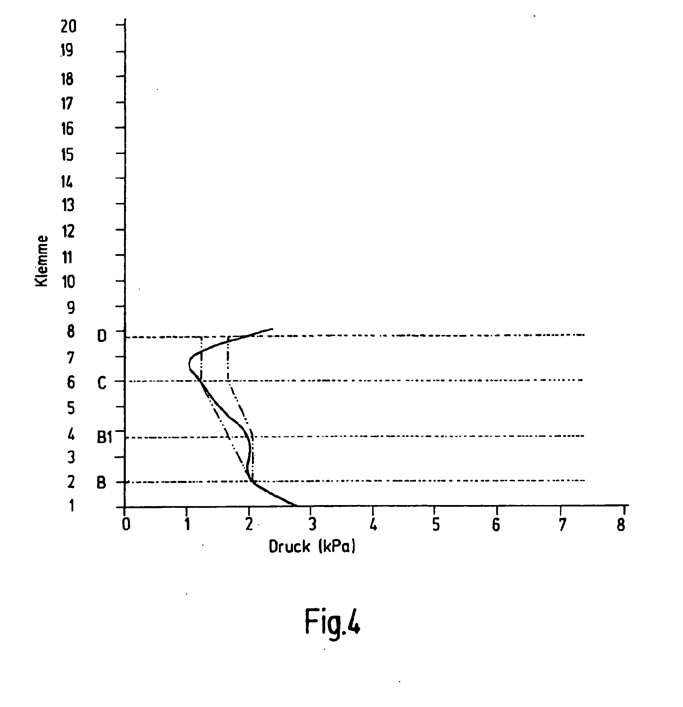

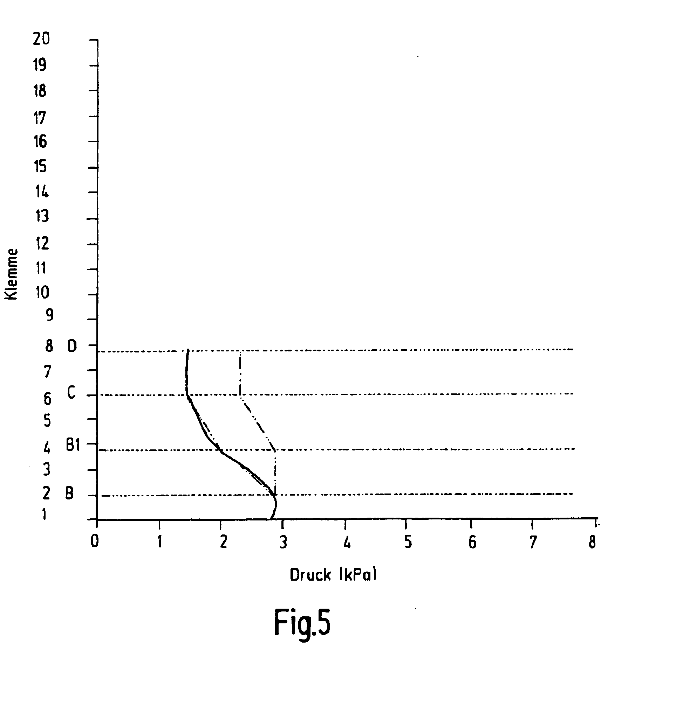

Den nachfolgenden Figuren 4 und 5 lassen sich des Weiteren Druckverläufe für den Über- und den Unterstrumpf entnehmen. Die Kompressionsmessung und Anbringung der Klemmen erfolgt dabei laut RAL-GZ 387, wobei für den Unterstrumpf (Figur 4) im Bereich zwischen B und B1 ein nach anfänglich geringem Druckabfall weitestgehend konstanter Kompressionsdruck besteht. Durch die doppelstrichpunktierten Linien wird der zulässige Bereich laut RAL-Norm (RAL-GZ 387) definiert. Bei dem Unterstrumpf handelt es sich dabei erkennbar nicht um einen Strumpf gemäß RAL-Norm (RAL-GZ 387), da dieser zwischen dem Messpunkt C und D den vorgeschriebenen Bereich verlässt. Der Darstellung für den Überstrumpf (Figur 5) lässt sich dagegen entnehmen, dass dieser Strumpf im Wesentlichen dem unteren Druckverlauf für einen Normstrumpf gemäß RAL-Norm (RAL-GZ 387) entspricht. Die Messung des Kompressionsdrucks erfolgt hierbei mittels eines Kompressionsprüfgerätes nach dem System Hohenstein (Hosy), wobei eine detaillierte Beschreibung des Prüfverfahrens sich aus dem Hohensteiner Forschungsbericht vom Januar 1982 und Phlebol und Proktol. 11: 34-41 (1982) entnehmen lässt.

Weitere Vorteile und Merkmale der Erfindung ergeben sich aus den übrigen Anmeldungsunterlagen.

Auf die vorstehend beschriebene Weise kann ein Kompressionsstrumpf oder Stützstrumpf bereitgestellt werden, der besonders gut zur Therapie von Leiden eingesetzt werden kann, bei denen offene Wunden und Hautläsionen durch eine Wundauflage innerhalb des Kompressionsbereichs überdeckt werden müssen.

## Patentansprüche

1. Kompressions- oder Stützstrumpf zum Anlegen an ein menschliches Bein umfassend einen ersten Unterstrumpf (10a) und einen ersten Überstrumpf (10b) mit je einem Schaftbereich (11a, 11b) und einem Fußbereich (12a, 12b), die übereinander anlegbar sind, wobei der Unterstrumpf (10a) im Bereich zwischen den Messstellen B und B1 einen kontinuierlichen Druckverlauf aufweist, und der Druck an der Messstelle B1 90-100% des Drucks an der Messstelle B entspricht, **dadurch gekennzeichnet, dass** im Schaftbereich (11) von Unter-(10a) und Überstrumpf (10b) ein in Umfangsrichtung verlaufendes Wellenmuster so eingstrickt ist, dass sich erhabene und weniger erhabene Zonen bilden.

2. Kompressions- oder Stützstrumpf nach Anspruch 1, **dadurch gekennzeichnet, dass** der Unter- (10a) und Überstrumpf (10b) Gestricke, vorzugsweise Rundgestricke sind.

3. Kompressions- oder Stützstrumpf nach Anspruch 1 oder 2,
**dadurch gekennzeichnet, dass** der Unterstrumpf (10a) an der Messstelle B1 von 93-100%, insbesondere von 95-100%, insbesondere von 97-100% des Drucks an der Messstelle B entspricht.

4. Kompressions- oder Stützstrumpf nach einem der vorangehenden Ansprüche, **dadurch gekennzeichnet, dass** der Überstrumpf (10b) ausgehend von der Messstelle B in Richtung der Hüfte des menschlichen Beins einen stufenlos degressiven oder stufenweise degressiven Druckverlauf aufweist und insbesondere der Überstrumpf (10b) ein Kompressionsstrumpf mit einem Druckverlauf gemäß RAL-GZ 387 ist.

5. Kompressions- oder Stützstrumpf nach einem der vorangehenden Ansprüche, **dadurch gekennzeichnet, dass** der Unterstrumpf (10a) im Bereich proximal Messstelle B1 einen stufenlos degressiven, stufenweise degressiven oder kontinuierlichen Druckverlauf aufweist.

6. Kompressions- oder Stützstrumpf nach einem der vorangehenden Ansprüche, **dadurch gekennzeichnet, dass** der Kompressionsdruck von Über- (10b) und Unterstrumpf (10a) zusammen *666,61-1999,83 Pa* (5-15 mm Hg) über dem Kompressionsdruck der Einzelstrümpfe (10a, 10b) liegt.

7. Kompressions- oder Stützstrumpf nach Anspruch 6, **dadurch gekennzeichnet, dass** die Muster von Unter- (10a) und Überstrumpf (10b) im übereinandergezogenen Zustand deckungsgleich sind.

8. Kompressions- oder Stützstrumpf nach einem der vorangehenden Ansprüche, **dadurch gekennzeichnet, dass** der Unterstrumpf (10a) eine Markierung zur Positionierung des Überstrumpfes (10b) beinhaltet.

9. Kompressions- oder Stützstrumpf nach Anspruch 8, **dadurch gekennzeichnet, dass** die Markierung durch einen Farb-, Textur- oder Musterwechsel im Unterstrumpf gebildet ist.

10. Kompressions- oder Stützstrumpf nach einem der vorangehenden Ansprüche, **dadurch gekennzeichnet, dass** der Unterstrumpf (10a) einen angeformten durch thermische Behandlung ausgebildeten Fersenbereich im Fußbereich (12a) aufweist.

11. Kompressions- oder Stützstrumpf nach einem der vorangehenden Ansprüche, **dadurch gekennzeichnet, dass** der Unter- (10a) und/oder der Überstrumpf (10b) wenigstens im Schaftbereich (11a, 11b) Fäden umfassen, bei denen ein elastischer Kernfaden mit einem Baumwollfasern enthaltenden Garn, insbesondere einen Stapelfasergarn umwickelt ist.

12. Kompressions- oder Stützstrumpf nach Anspruch 11, **dadurch gekennzeichnet, dass** das Baumwollfasern enthaltende Garn 70-90 % Baumwolle und 10-30 % einer Algen enthaltenden Faser umfasst.

## Claims

1. Compression or support stocking for application to a human leg, comprising a first understocking (10a) and a first overstocking (10b), each with a shaft section (11a, 11b) and a foot section (12a, 12b), applicable over each other, wherein the understocking (10a) has a continuous pressure progression in the area between measuring points B and B1, and the pressure at measuring point B1 amounts to 90%-100% of the pressure at measuring point B, **characterized in that** in the shaft section (11) of the understocking (10a) and the overstocking (10b) a wave pattern running in a circumferential direction is knitted so as to form raised and less raised zones.

2. Compression or support stocking according to claim 1, **characterized in that** the understocking (10a) and the overstocking (10b) are knit, preferably circular knit, fabric.

3. Compression or support stocking according to claim 1 or 2, **characterized in that** the pressure at measuring point B1 in the understocking (10a) corresponds to between 93% and 100%, in particular between 95% and 100% and in particular between 97% and 100% of the pressure at measuring point B.

4. Compression or support stocking according to one of the preceding claims, **characterized in that** the overstocking (10b) has a continuously degressive or gradually degressive pressure progression starting from measuring point B and progressing towards the hip of the human leg and that in particular the overstocking (10b) is a compression stocking with a a pressure progression in accordance with RAL-GZ 387.

5. Compression or support stocking according to one of the preceding claims, **characterized in that** the understocking (10a) has a continuously degressive or gradually degressive pressure progression in the area proximal measuring point B1.

6. Compression or support stocking according to one of the preceding claims, **characterized in that** the joint compression pressure of the overstocking (10b) and the understocking (10a) is 666,61-1999,83 Pa (5-15 mm Hg) higher than the compression pressure of the individual stockings (10a, 10b).

7. Compression or support stocking according to claim 6, **characterized in that** the patterns of understocking (10a) and overstocking (10b) are congruent when the stockings are worn over each other.

8. Compression or support stocking according to one of the preceding claims, **characterized in that** the understocking (10a) has a marking for the positioning of the overstocking (10b).

9. Compression or support stocking according to claim 8, **characterized in that** the marking consists of a change of color, texture or pattern in the understocking.

10. Compression or support stocking according to one of the preceding claims, **characterized in that** the understocking (10a) has an integrally formed heel section developed through thermal treatment in the foot section (12a).

11. Compression or support stocking according to one of the preceding claims, **characterized in that** the understocking (10a) and/or the overstocking (10b) comprises threads, at least in the shaft sections (11a, 11b), where an elastic core thread is enwound with a cotton fiber containing yarn, in particular a staple fiber yarn.

12. Compression or support stocking according to claim 11, **characterized in that** the cotton fiber containing yarn contains 70%-90% cotton and 10-13% algae containing fibers.

## Revendications

1. Bas de compression ou de contention à mettre sur une jambe humaine comprenant un premier bas de dessous (10a) et un premier bas de dessus (10b) dotés respectivement d'une zone de tige (11a, 11b) et d'une zone de pied (12a, 12b) pouvant être mises l'une sur l'autre, le bas de dessous (10a) comprenant dans la zone située entre les points de mesure B et B1 un profil de pression continu, et la pression au point de mesure B1 correspondant à 90 à 100 % de la pression au point de mesure B, **caractérisé en ce qu'**un motif ondulé s'étendant dans la direction circonférentielle est tricoté dans la zone de tige (11) du bas de dessous (10a) et du bas de dessus (10b) de manière à ce que des zones en relief et moins en relief se forment.

2. Bas de compression ou de contention selon la revendication 1, **caractérisé en ce que** le bas de dessous (10a) et le bas de dessus (10b) sont des tricots, de préférence des tricots tubulaires.

3. Bas de compression ou de contention selon la revendication 1 ou 2, **caractérisé en ce que** le bas de dessous (10a) au point de mesure B1 correspond à 93 à 100 %, en particulier à 95 à 100 %, en particulier à 97 à 100 % de la pression au point de mesure B.

4. Bas de compression ou de contention selon l'une quelconque des revendications précédentes, **caractérisé en ce que** le bas de dessus (10b) présente en partant du point de mesure B en direction de la hanche de la jambe humaine un profil de pression dégressif en continu ou dégressif par étapes et en particulier le bas de dessus (10b) est un bas de compression doté d'un profil de pression conformément à RAL-GZ 387.

5. Bas de compression ou de contention selon l'une quelconque des revendications précédentes, **caractérisé en ce que** le bas de dessous (10a) présente dans la zone à proximité du point de mesure B1 un profil de pression dégressif en continu, dégressif par étapes ou continu.

6. Bas de compression ou de contention selon l'une quelconque des revendications précédentes, **caractérisé en ce que** le cumul de la pression de compression du bas de dessus (10b) et de celle du bas de dessous (10a) est supérieur de 666,61 à 1999,83 Pa (5-15 mm Hg) à la pression de compression de chaque bas (10a, 10b).

7. Bas de compression ou de contention selon la revendication 6, **caractérisé en ce que** les motifs du bas de dessous (10a) et du bas de dessus (10b) coïncident lorsque les bas sont enfilés et superposés.

8. Bas de compression ou de contention selon l'une quelconque des revendications précédentes, **caractérisé en ce que** le bas de dessous (10a) comporte une marque de positionnement du bas de dessous (10b).

9. Bas de compression ou de contention selon la revendication 8, **caractérisé en ce que** la marque est formée par un changement de couleur, de texture ou de motif dans le bas de dessous.

10. Bas de compression ou de contention selon l'une quelconque des revendications précédentes, **caractérisé en ce que** le bas de dessous (10a) comprend dans la zone de pied (12a) une zone de talon formée par traitement thermique.

11. Bas de compression ou de contention selon l'une quelconque des revendications précédentes, **caractérisé en ce que** le bas de dessous (10a) et/ou le bas de dessus (10b) comprennent au moins dans la zone de tige (11a, 11b) des fils pour lesquels une âme élastique est enroulée avec un fil contenant des fibres de coton, en particulier un fil à fibre discontinue.

12. Bas de compression ou de contention selon la revendication 11, **caractérisé en ce que** le fil contenant des fibres de coton comprend des fibres contenant 70 à 90 % de coton et 10 à 30 % d'une algue.
